Europäisches Patentamt

(19) **European Patent Office**

Office européen des brevets

(11) Veröffentlichungsnummer: **0 045 818**
**B1**

(12) # EUROPÄISCHE PATENTSCHRIFT

(45) Veröffentlichungstag der Patentschrift:
**22.05.85**

(51) Int. Cl.⁴: **A 61 F 2/56**

(21) Anmeldenummer: **80104775.4**

(22) Anmeldetag: **13.08.80**

(54) **Handprothese.**

(43) Veröffentlichungstag der Anmeldung:
**17.02.82 Patentblatt 82/7**

(45) Bekanntmachung des Hinweises auf die Patenterteilung:
**22.05.85 Patentblatt 85/21**

(84) Benannte Vertragsstaaten:
**AT BE CH FR GB IT LI LU NL SE**

(56) Entgegenhaltungen:
**DE - A - 2 433 710**
**DE - C - 301 803**
**DE - C - 303 587**
**DE - C - 303 696**
**DE - C - 320 702**
**DE - C - 365 455**
**DE - C - 746 023**
**FR - A - 481 317**
**FR - A - 483 491**
**FR - A - 490 594**
**FR - A - 545 837**
**US - A - 2 542 316**
**US - A - 2 545 452**
**US - A - 2 549 074**

(73) Patentinhaber: **Grau, Hermann, Leinweg 25,**
**D-7070 Schwäbisch Gmünd-Lindach (DE)**

(72) Erfinder: **Engelhardt, Achim, Dr.,**
**Donnersbergstrasse 42, D-6000 Frankfurt / Main (DE)**
Erfinder: **Franke, Horst, Prof. Dipl.-Ing., Weilerstrasse 23,**
**D-7080 Aalen (DE)**
Erfinder: **Strobel, Peter, Ing.-grad, Treppacher Strasse 1,**
**D-7070 Aalen 15 (DE)**

(74) Vertreter: **Hoeger, Stellrecht & Partner,**
**Uhlandstrasse 14c, D-7000 Stuttgart 1 (DE)**

**Beschreibung**

Die Erfindung betrifft eine Handprothese gemäss dem Oberbegriff des Patentanspruchs 1.

Bei einer bekannten Handprothese dieser Art (DE-C-3 035 587) erfolgt das Schliessen der Finger, also deren Beugung, mit Hilfe von künstlichen «Sehnen», die als Zugseile ausgebildet und auf im Handtellerbereich vorgesehene Wellen aufwickelbar sind. Die dem Öffnen, d.h. dem Strecken der Finger dienenden Gelenkfedern sind bei der bekannten Handprothese als Zugfedern in Gestalt von Schraubenfedern ausgebildet.

Bei einer weiteren bekannten Handprothese (DE-C-320 702) erfolgt das Schliessen der Finger wiederum über Zugseile, die über Rollen laufen, während die Streckung der Finger mit Hilfe von Spiralfedern vorgenommen wird, die an den Gelenkstellen angeordnet sind.

Schliesslich ist es auch bekannt (DE-C-365 455), zum Schliessen der Finger Organe beliebiger Art zu verwenden und die Streckung der Finger durch sich verzweigende Zugseile zu bewirken.

Bei den bekannten Handprothesen sind die Zugseile auch bei gestreckten Fingern gespannt. Dies führt dazu, dass beim Schliessen der Finger, zumindest im Leerlauf, d.h. ohne Auftreffen auf ein Hindernis, sowie beim Öffnen eine Bewegung abläuft, die dem entsprechenden Bewegungsablauf bei einer natürlichen menschlichen Hand nicht entspricht. Bei der natürlichen menschlichen Hand werden nämlich beim Beugen der Finger stets zuerst die äusseren Fingerglieder verschwenkt, während die Schwenkbewegung des mit dem Handteller verbundenen Fingergliedes zuletzt erfolgt. Bei den bekannten Handprothesen ist der Bewegungsablauf genau umgekehrt. Abgesehen davon, dass diese unterschiedliche Kinematik den Gebrauch der Handprothese, insbesondere beim Ergreifen schmaler und dünner Gegenstände beeinträchtigt, ist an diesem, vom natürlichen verschiedenen Bewegungsablauf sofort erkennbar, dass es sich um eine Prothese handelt, was für den Träger der Prothese unangenehm sein kann.

Es ist Aufgabe der Erfindung, eine gattungsgemässe Handprothese so auszubilden, dass beim Schliessen der Finger zuerst jeweils die vorderen, vom Handkörper entfernter liegenden Fingerglieder gebeugt werden, bevor sich die hieran anschliessenden, näher am Handkörper gelegenen Fingerglieder entsprechend verschwenken, während sich beim Öffnen der Handprothese die Bewegungen der Fingerglieder in umgekehrter Reihenfolge vollziehen sollen.

Diese Aufgabe wird durch die Merkmale im Kennzeichnungsteil des Patentanspruchs 1 gelöst.

Die nachstehende Beschreibung einer bevorzugten Ausführungsform der Erfindung dient im Zusammenhang mit beiliegender Zeichnung der weiteren Erläuterung. Es zeigen:

Fig. 1 eine Ansicht der Innenseite einer Handprothese und

Fig. 2 eine gegenüber Fig. 1 vergrösserte Schnittdarstellung in Seitenansicht eines Langfingers bei einer gegenüber Fig. 1 geringfügig abgewandelten Ausführungsform.

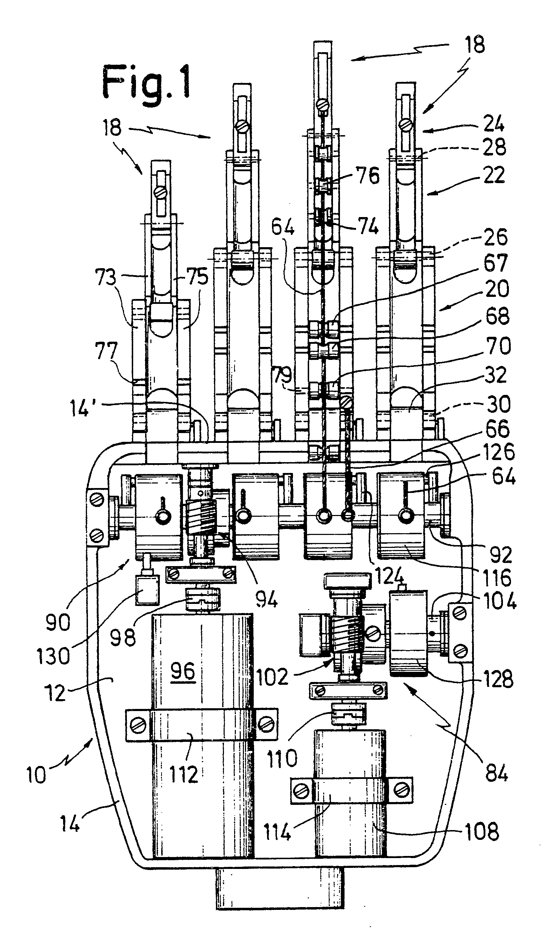

Die Fig. 1 zeigt mit Blick auf die Handinnenseite einen Handkörper 10, der einen im wesentlichen ebenen, den Handrücken bildenden Boden 12 aufweist. Auf einer Umfangswand 14 des Handkörpers 10 ist ein (nicht dargestellter) Deckel befestigbar, welcher die Handinnenseite bildet. Der Handkörper 10 und der Deckel sind auf Kunststoff im Spritzgussverfahren hergestellt, wobei der Handkörper 10 mit Boden 12 naturgetreu dem menschlichen Handrücken und der Deckel naturgetreu der Handinnenfläche nachgebildet sein kann.

Am vorderen, gemäss Fig. 1 oberen Umfangswandteil 14' des Handkörpers 10 sind in seitlichem Abstand voneinander insgesamt vier als Ganzes mit 18 bezeichnete Langfinger angeordnet, deren Länge an die Fingerlänge einer menschlichen Hand angepasst ist.

Im Ausführungsbeispiel sind die Langfinger durch jeweils drei Fingerglieder, nämlich ein Handglied 20, ein Mittelglied 22 und ein Nagelglied 24 gebildet, die durch Schwenkachsen 26 und 28 gelenkig miteinander verbunden sind.

Das Handglied 20 sitzt auf einer Schwenkachse 30, die ihrerseits in einem an dem oberen Umfangswandteil 14' angeformten Haltesteg 32 befestigt ist.

Den Langfingern 18 ist ein (nicht dargestellter) Daumen zugeordnet, welcher an der Oberseite des Deckels in einer Anordnung vorgesehen ist, die derjenigen des Daumens der menschlichen Hand im wesentlichen entspricht.

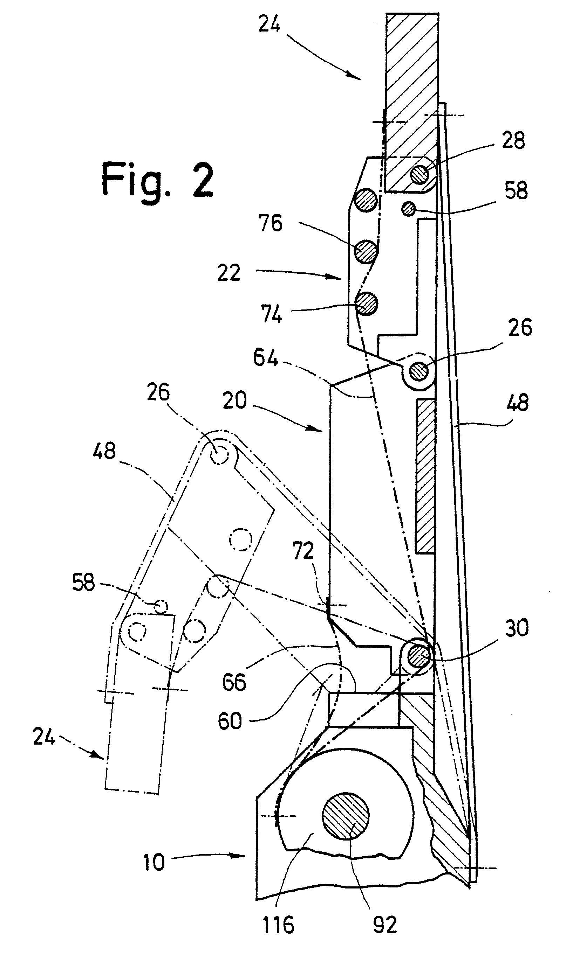

Die durch die Schwenkachsen 26, 28, 30 gebildeten Gelenke der Langfinger sind an ihren Rückseiten jeweils von lediglich einem «Gelenkfeder» 48 genannten Rückstellelement in Gestalt eines hochelastischen Gummibandes von geringer Federkraft-Federweg-Hysterese überbrückt. Wie aus Fig. 2 ersichtlich, ist die Gelenkfeder 48 einerseits am Handkörper 10 und andererseits am Nagelglied 24 befestigt, sie überspannt also das Mittelglied 22 frei.

Die Gelenkfedern 48 haben das Bestreben, die Finger ständig in gestreckter Stellung zu halten.

Wie Fig. 2 zeigt, ist das Nagelglied 24 unter die Norm des die Gelenkfeder 48 bildenden Gummibandes relativ zum Mittelglied 22 maximal in eine Stellung verschwenkbar, die durch einen am Mittelglied angeordneten Anschlag 58 bestimmt ist. Ebenso ist das Handglied 20 in eine durch eine Anschlagfläche 60 bestimmte Endlage zu bringen. Zum Beugen jedes Fingers dienen zwei Zugseile 64 und 66, die sich an der Innenseite der Finger entlang erstrecken. Das Zugseil 64 ist am Handglied 20 durch Seilführungsrollen 67, 68 70 (nur in Fig. 1 dargestellt) und am Mittelglied 22 durch Seilführungsrollen 74, 76 geführt und am Nagelglied verankert. Die Fingerglieder sind durch im Querschnitt U-förmige Teile aus Kunststoff hergestellt. Zur Montage der Seilführungsrollen sind diese Teile an ihren Schenkeln 73, 75 mit nach innen gerichteten Schlitzen 77 versehen, in die jeweils eine Achse 79 der Seilrollen eindrückbar und in einem erweiterten Schlitzteil verrastbar ist.

Das andere Zugseil 66 ist am unteren Ende 72 des Handgliedes 20 befestigt.

Die durch einen Kunstfaserfaden gebildeten Zugseile 64, 66 sind durch den Umfangswandteil 14' hindurch zu einer als Ganzes mit 84 bzw. 90 bezeichneten Betätigungsvorrichtung geführt, die innerhalb des Handkörpers 10 angeordnet ist. Die Betätigungsvorrichtung 84 dient der Betätigung des Daumens, die Betätigungsvorrichtung 90 der Betätigung der Langfinger. Die Betätigungsvorrichtung 90 weist eine in den seitlichen Umfangswandteilen des Handkörpers 10 drehbar gelagerte Antriebswelle 92 auf, die über ein Schneckengetriebe 94 in beiden Richtungen selbsthemmend verdrehbar ist. Das Schneckengetriebe 94 wird durch einen umpolbaren Gleichstromgetriebemotor 96 unter Zwischenschaltung einer Kreuzschieberkupplung 98 angetrieben. Zwischen Antriebswelle 92 und Schneckengetriebe 94 ist eine durch eine Rutschkupplung gebildete Überlastsicherung eingebaut.

Die Betätigungsvorrichtung 84 des Daumens weist einen analogen Aufbau auf. Sie umfasst eine durch ein Schneckengetriebe 102 antreibbare Antriebswelle 104 und eine zwischen diesen beiden Teilen gelegene Rutschkupplung. Das Schneckengetriebe 102 wird durch einen umpolbaren Gleichstromgetriebemotor 108 angetrieben. Zwischen Schneckengetriebe und Motor liegt wiederum eine Kreuzschieberkupplung 110.

Aus Fig. 1 und 2 geht hervor, dass die Antriebswelle 92 im wesentlichen zum Boden 12 des Handkörpers 10 sowie zu den Schwenkachsen 26, 28, 30 der Langfinger parallel verläuft. Die Antriebswelle 104 erstreckt sich zur Antriebswelle 92 ebenfalls im wesentlichen parallel. Zu diesen Antriebswellen verlaufen die Wellen der Motoren 96, 108 im wesentlichen senkrecht. Die Motoren sind mittels Befestigungslaschen 112 bzw. 114 am Boden 12 des Handkörpers auswechselbar gehalten.

Auf der Antriebswelle 92 ist je Langfinger eine Wickeltrommel 116 verdrehbar angeordnet, an deren Umfang die Enden der beiden Zugseile 64, 66 des betreffenden Langfingers befestigt sind. Die Wickeltrommeln bilden Federgehäuse, in denen jeweils eine spiralförmige Triebfeder angeordnet ist. Die Triebfeder ist einerseits am Umfang des Federgehäuses und andererseits am Umfang der Antriebswelle 92 in bekannter Weise verankert.

Jeder Wickeltrommel ist am Umfang der Antriebswelle 92 ein Anschlag 124 zugeordnet. Alle Anschläge 124 sitzen auf einer gemeinsamen Mantellinie der Antriebswelle 92. Jeder Anschlag wirkt mit einem an der zugehörigen Wickeltrommel befestigten Gegenanschlag 126 zusammen.

Die Wickeltrommeln 116 sind derart axial verschiebbar auf der Antriebswelle 92 gelagert, dass sie sich relativ zu dem ihnen zugeordneten Anschlag 124 zum Vorspannen der Triebfedern entgegen der Drehrichtung der Antriebswelle 92 beim Schliessen der Hand verdrehen lassen.

Eine Zwangsmitnahme der Finger durch die Zugseile 64, 66 tritt dann auf, wenn die Anschläge 124 der Antriebswelle 92 auf die Gegenanschläge 126 der Wickeltrommeln auflaufen. Von diesem Zeitpunkt an werden die Triebfedern kurzgeschlossen, indem die Wickeltrommeln durch die Antriebswelle formschlüssig angetrieben werden. Dadurch wird ein Kraftgriff erzeugt, und die Hand kann nicht mehr von aussen geöffnet werden. Beim Abschalten des Antriebsmotors bleibt dabei die Griffkraft infolge des selbsthemmenden Schneckengetriebes 94 erhalten. Dabei ist die Tragfähigkeit der Langfinger nur durch die Haltbarkeit der Zugseile oder des Schneckengetriebes begrenzt.

Das motorische Öffnen der Finger erfolgt durch Umpolen der Gleichstrom-Getriebemotoren 96, 108, wobei die Fingerrückführung ausschliesslich durch die Gelenkfedern 48 bewerkstelligt wird, da die Zugseile 64, 66 keine Rückstellkräfte übertragen können.

Für viele Greifbewegungen ist die Einzelbeweglichkeit der Langfinger nicht erforderlich. Es ist daher auch möglich, die Zugseile von jeweils zwei Fingern, und zwar Zeige- und Mittelfinger sowie Ring- und kleiner Finger, auf eine gemeinsame Wickeltrommel aufzuwickeln. In diesem Falle können zwei Wickeltrommeln nebst Triebfedern eingespart werden.

Die Betätigungsvorrichtung 84 des Daumens ist ebenfalls mit einer Wickeltrommel 128 zum Aufwickeln der betreffenden Zugseile 64, 66 ausgestattet, die jedoch im Gegensatz zur vorbeschriebenen Konstruktion auf der Antriebswelle 104 drehfest angeordnet ist. Dies ist deshalb notwendig, weil der Daumen bei Greifbewegungen ein Widerlager zu den Langfingern bilden muss und nicht weggedrückt werden darf.

Mit 130 ist ein Endschalter bezeichnet, dem am Umfang der gemäss Fig. 1 links aussen liegenden Wickeltrommel 116 ein nicht gezeigter Schaltnokken zugeordnet ist. Dieser betätigt den Endschalter zur Abschaltung des Getriebemotors 96, sobald die Langfinger ihre maximale Endstellung beim Öffnen erreicht haben. Ein weiterer, nicht gezeichneter Endschalter, der durch einen nicht gezeichneten Schaltnocken auf der Wickeltrommel 128 betätigt wird, schaltet den Getriebemotor 108 des Daumens ab, sobald letzterer seine Endstellung beim Öffnen erreicht hat. Erreichen die Langfinger und der Daumen ihre maximale Schliessstellung, bleiben die Getriebemotoren stehen. Hierbei verbleiben sie unter Strom oder sie werden, vom Haltestrom gesteuert, abgeschaltet.

Die Getriebemotoren 96, 108 können kontaktgesteuert oder myo-elektrisch ansteuerbar sein.

Die Länge der beiden Zugseile 64, 66 ist aufeinander derart abgestimmt, dass bei gestrecktem Finger das Zugseil 66 spannungslos ist. Bei Ingangsetzen der Wickeltrommel 116 bzw. 128 wird zunächst das Nagelglied 24 und daran anschliessend das Mittelglied 22 verschwenkt. Erst im Verlauf von dessen Schwenkbewegung wird durch das nun unter Spannung geratene Zugseil 66 das Handglied 20 gebeugt. Die Bewegungsfolge der Fingerglieder lässt sich durch eine entsprechende Bemessung der Länge des Zugseiles 66 einstellen.

Der Bewegungsverlauf der Fingerglieder bei der. Rückführung des Fingers in seine gestreckte Stellung wird damit ebenfalls ausschliesslich durch die Zugseile 64, 66 gesteuert, so dass es ausreicht, das Gummiband der Gelenkfeder 48 lediglich an dessen Nagelglied 24 zu verankern.

Die geschilderte Konstruktion bietet die Möglichkeit, Zugseile verschiedener Zugfestigkeit zu verwenden, so dass z. B. zur Betätigung des Handgliedes 20 ein Zugseil 66 verwendet werden kann, das es ermöglicht, auch extrem schwere Lasten zu tragen.

Die Zugfestigkeit des zur Betätigung des am Handkörper 10 angelenkten Fingergliedes 20 dienenden Zugseiles 66 kann grösser als diejenige des anderen Zuggliedes 64 sein.

Die beiden Vorrichtungen 84, 90 zum Aufwickeln des Zugseiles beim Beugen der Finger können manuell verstellbar ausgebildet sein.

Schliesslich kann in bekannter Weise mindestens ein zum Beispiel mechanisch oder akustisch arbeitendes Rückmeldeelement zur Signalisierung des erfolgten Ergreifens eines Gegenstandes vorgesehen sein, das mindestens mittelbar durch den die als Getriebemotoren ausgebildete Betätigungsvorrichtung 96 der Langfinger steuernden Strom ansteuerbar ist.

**Patentansprüche**

1. Handprothese mit an einem Handkörper (10) angelenkten Fingern (18) aus mindestens zwei, durch ein Fingergelenk (26, 28) miteinander verbundenen Fingergliedern (20, 22, 24), deren Schwenkbarkeit durch Anschläge (58) begrenzt ist, mit undehnbaren Zugseilen (64, 66), von denen jeweils mindestens eines an der Innenseite der Finger in deren Längsrichtung verläuft und mit einem Ende an einem vorderen Fingerglied (24) befestigt ist, mit einer im Handkörper angeordneten Vorrichtung (84, 90) zum Aufwickeln des anderen Endes des Zugseiles beim Beugen der Finger und mit dem Strecken der Finger dienenden, an deren Rückseite angeordneten, vorgespannten und dehnbaren Gelenkfedern (48), die jeweils mehrere Fingergelenke übergreifen und mit ihren Enden an den vorderen Fingergliedern und dem Handkörper festgelegt sind, dadurch gekennzeichnet, dass mit dem am Handkörper (10) angelenkten Fingerglied (20) und mit dem an diesem angelenkten, weiteren Fingerglied (22) eines Fingers (18) je ein Zugseil (64 bzw. 66) verbunden ist, deren Länge so aufeinander abgestimmt ist, dass bei gestrecktem Finger (18) das mit dem am Handkörper angelenkten Fingerglied verbundene Zugseil (66) spannungslos ist.

2. Handprothese nach Anspruch 1, dadurch gekennzeichnet, dass die Zugfestigkeit des zur Betätigung des am Handkörper (10) angelenkten Fingergliedes (20) dienenden Zuggliedes (66) grösser ist als diejenige des anderen Zuggliedes (64).

3. Handprothese nach Anspruch 1 oder 2, dadurch gekennzeichnet, dass die Zugglieder (64, 66) aus einem Kunstfaserseil bestehen.

4. Handprothese nach Anspruch 1, 2 oder 3, dadurch gekennzeichnet, dass die Fingerglieder (20, 22 24) durch Formteile aus Kunststoff gebildet sind, in deren Schenkeln (73, 75) zur Anordnung von Rollenachsen (79) vom Schenkelrand sich nach innen erstreckende Schlitze (77) angeordnet sind, in welche die Rollenachsen (79) mit ihren aus Rollen (74, 76) herausragenden Endstücken eindrückbar sind.

5. Handprothese nach einem der Ansprüche 1 bis 4, gekennzeichnet durch zwei im als Aufnahmegehäuse ausgebildeten und durch einen insbesondere abnehmbaren Deckel verschliessbaren Handkörper (10) angeordnete Betätigungsvorrichtungen (84, 90), von denen die eine zur Betätigung von Langfingern (18) und die andere zur Daumenbetätigung dient.

6. Handprothese nach Anspruch 5, dadurch gekennzeichnet, dass beide Betätigungsvorrichtungen (84, 90) manuell verstellbar sind.

7. Handprothese nach Anspruch 5, dadurch gekennzeichnet, dass beide Betätigungsvorrichtungen (84, 90) durch jeweils eine elektrische Antriebsvorrichtung (96, 108) antreibbar sind.

8. Handprothese nach Anspruch 7, dadurch gekennzeichnet, dass die Antriebsvorrichtungen (96, 108) myo-elektrisch ansteuerbar sind.

9. Handprothese nach Anspruch 7, dadurch gekennzeichnet, dass die Antriebsvorrichtungen (96, 108) kontaktgesteuert sind.

10. Handprothese nach Anspruch 6 oder 7, dadurch gekennzeichnet, dass die zur Langfingerbetätigung dienende Betätigungsvorrichtung (90) die folgenden bekannten Merkmale aufweist:

a) je Langfinger (18) ist eine Wickeltrommel (116) vorgesehen, an deren Umfang das Zugglied (64, 66) des zugeordneten Langfingers (18) aufwickelbar ist;

b) die Wickeltrommeln (116) sind koaxial zueinander angeordnet und durch ein gemeinsames Antriebsglied verdrehbar;

c) die Wickeltrommeln (116) sind gegen die Wirkung einer diesen zugeordneten Rückstellfeder um einen Drehwinkel relativ zueinander verdrehbar, der je Wickeltrommel (116) durch zwei Anschläge (124, 126) definiert ist, von denen der eine am Antriebsglied und der andere an der Wickeltrommel angeordnet ist.

11. Handprothese nach Anspruch 10, bei der die zur Langfingerbetätigung dienende Betätigungsvorrichtung (90) durch eine kontakt- oder myoelektrisch gesteuerte Antriebsvorrichtung (96) antreibbar ist, dadurch gekennzeichnet, dass die Wickeltrommeln (116) in bekannter Weise auf einem gemeinsamen Träger angeordnet sind, dass der Träger eine verdrehbare Antriebswelle (92) bildet, die über ein Schneckengetriebe (94) durch die Antriebsvorrichtung (96) antreibbar ist, dass die Wickeltrommeln (116) durch auf der Antriebswelle (92) mindestens drehbar angeordnete Federgehäuse und deren Rückstellfedern durch in diesen angeordnete spiralförmige Triebfedern

7  0 045 818  8

(Aufzugfedern) gebildet sind, die mit ihrem einen Ende am Umfang des Federgehäuses und mit ihrem anderen Ende am Umfang der Antriebswelle (92) verankert sind, und dass am Umfang der letzteren je Federgehäuse ein Anschlag (124) vorgesehen ist, an welchem das zugeordnete Federgehäuse mit einem Gegenanschlag (126) unter der Wirkung einer durch die Triebfeder erzeugten Anpresskraft ahliegt.

12. Handprothese nach Anspruch 11, dadurch gekennzeichnet, dass die Federgehäuse (116) zum Vorspannen ihrer Triebfeder auf der Antriebswelle (92) axial verstellbar angeordnet sind.

13. Handprothese nach Anspruch 11 oder 12, dadurch gekennzeichnet, dass zwischen Schneckengetriebe (94) und Antriebswelle (92) eine Überlastsicherung, insbesondere eine einstellbare Rutschkupplung, zwischengeschaltet ist.

14. Handprothese nach einem der Ansprüche 7 bis 13, dadurch gekennzeichnet, dass der Daumen auf der Aussenseite des Deckels des den Handkörper (10) bildenden Aufnahmegehäuses, insbesondere mit der Daumenwurzel verdrehbar und feststellbar, angeordnet ist, und dass das durch den Deckel hindurchgeführte Zugglied (64) auf eine Wickeltrommel (128) der zur Betätigung des Daumens dienenden Betätigungsvorrichtung (84) aufwickelbar ist, die ihrerseits drehfest auf einer Antriebswelle (104) sitzt, welche über ein durch eine Überlastsicherung, insbesondere eine Rutschkupplung, abgesichertes Schneckengetriebe (102) durch die elektrische Antriebsvorrichtung (108) antreibbar ist.

15. Handprothese nach Anspruch 14, dadurch gekennzeichnet, dass die Antriebsvorrichtungen (96, 108) der beiden Betätigungsvorrichtungen (84, 90) durch einen umpolbaren, insbesondere als Kleinstmotor mit geringer Leistung ausgelegten Gleichstrom-Getriebemotor gebildet sind.

16. Handprothese nach Anspruch 15, dadurch gekennzeichnet, dass zwischen den Antriebsvorrichtungen (96, 108) und den diesen nachgeschalteten Schneckengetrieben (94, 102) jeweils eine winkelbewegliche Kupplung, insbesondere Kreuzschieberkupplung (98, 110) angeordnet ist.

17. Handprothese nach einem der Ansprüche 14 bis 16, dadurch gekennzeichnet, dass die Antriebswellen (92, 104) beider Betätigungsvorrichtungen (84, 90) in dem den Handkörper (10) bildenden Aufnahmegehäuse zueinander im wesentlichen achsparallel und zu diesem senkrecht, jedoch zueinander gleichfalls im wesentlichen achsparallel die Getriebemotoren beider Antriebsvorrichtungen (96, 108) angeordnet sind.

18. Handprothese nach einem der voranstehenden Ansprüche, dadurch gekennzeichnet, dass in bekannter Weise mindestens ein zum Beispiel mechanisch oder akustisch arbeitendes Rückmeldeelement zur Signalisierung des erfolgten Ergreifens eines Gegenstandes vorgesehen ist, das mindestens mittelbar durch den die als Getriebemotoren ausgebildete Betätigungsvorrichtung (96) der Langfinger steuernden Strom ansteuerbar ist.

**Claims**

1. Artificial hand with fingers (18) articulated on a hand body (10), the fingers comprising at least two finger members (20, 22, 24), which are connected to one another by means of a finger joint (26, 28), the pivotability of which is limited by stops (58), with non-extendable traction cords (64, 66), of which in each case at least one runs along the inner side and in the longitudinal direction of the finger and is secured at one end to a front finger member (24), with a device (84, 90) disposed in the hand body for winding up the other end of the traction cord when the finger bends and with pretensioned and extendable joint springs (48) which are disposed on the backs of the fingers for stretching the latter, and which extend in each case over a plurality of finger joints and are secured at their ends to the front finger members and the hand body, caracterised in that one traction cord (64) is connected to the finger member (20), which is articulated on the hand body (10) and another traction cord (66) is connected to the further finger member (22) of a finger (18) which is articulated on the finger member (20), the lengths of the traction cords being dimensioned with respect to each another, so that the traction cord (66), which is connected to the finger member articulated on the hand body is without tension when the finger (18) is stretched.

2. Artificial hand according to claim 1, characterised in that the tensile strength of the traction member (66) for actuating the finger member (20) articulated on the hand body (10) is greater than that of the other traction member (64).

3. Artificial hand according to claim 1 or 2, characterised in that the traction members (64, 66) consist of a synthetic fibre cord.

4. Artificial hand according to claim 1, 2 or 3, characterised in that the finger members (20, 22, 24) are made of moulded parts of synthetic material, in the sides (7, 75) of which are disposed slots (77) extending inwards from the edge of the sides for accomodating roller axles (79), into which slots the roller axles (79) can be pressed with their end parts projecting from rollers (74, 76).

5. Artificial hand according to one of the claims 1 to 4, characterised by two actuating mechanisms (84, 90), disposed in the hand body (10), which is designed as a receiving housing and which can be closed by means of a cover, which in particular can be removed, one of the actuating mechanisms operating the fingers (18) and the other operating the thumb.

6. Artificial hand according to claim 5, characterised in that both actuating mechanisms (84, 90) can be manually adjusted.

7. Artificial hand according to claim 5, characterised in that both actuating mechanisms (84, 90) can be driven in each case by an electrical drive mechanism (96, 108).

8. Artificial hand according to claim 7, characterised in that the drive mechanisms (96, 108) can be myo-electrically controlled.

9. Artificial hand according to claim 7, characterised in that the drive mechanisms (96, 108) can be contact-controlled.

10. Artificial hand according to claim 6 or 7, characterised in that the actuating mechanism (90) for operating the fingers has the following features:

a) each finger (18) is provided with a winding drum (116), around the periphery of which the traction member (64, 66) of the associated finger (18) can be wound;

b) the winding drums (116) are disposed coaxially in relation to one other and can be rotated by means of a common drive member;

c) the winding drums (116) can be rotated relative to one another – to counteract the effect of a return spring which is associated with the winding drums – through an angle of rotation, which in the case of each winding drum (116) is limited by two stops (124, 126), of which one is disposed on the drive member and the other on the winding drum.

11. Artificial hand according to claim 10, in which the actuating mechanism (90) for operating the fingers can be driven by a contact or myo-electrically controlled drive mechanism (96), characterised in that the winding drums (116) are disposed in a known manner on a common support, the support forms a rotatable drive shaft (92), which can be driven via a worm drive (94) by a drive mechanism (96), the winding drums (116) are formed by spring housings disposed at least rotatably on the drive shaft (92) and their return springs are formed by spiral-shaped driving springs (winding springs), which are disposed within the spring housings and which are secured at one end to the periphery of the spring housing and at the other end to the periphery of the drive shaft (92), and one stop (124) is provided on the periphery of the latter per spring housing, against which stops the associated spring housing rests with an opposing stop (126) under the action of a contact pressure produced by the driving spring.

12. Artificial hand according to claim 11, characterised in that the spring housings (116) are disposed so that they can be axially adjusted on the drive shaft (92) for the pretensioning of their driving springs.

13. Artificial hand according to claim 11 or 12, characterised in that an overload safety mechanism, more particularly an adjustable sliding coupling, is interposed between the worm gear (94) and the drive shaft (92).

14. Artificial hand according to any one of the claims 7 to 13, characterised in that the thumb is disposed on the outside of the cover of the receiving housing forming the hand body (10), in particular so that it can be rotated with and secured to the thumb root, the traction member (64) which passes through the cover can be wound onto a winding drum (128) of the actuating mechanism for actuating the thumb, the actuating mechanism being mounted rotationally rigid on a drive shaft (104), which can be driven by the electrical drive mechanism (108) via a worm gear (102) which is protected by an overload safety mechanism, more particularly a sliding coupling.

15. Artificial hand according to claim 14, characterised in that the drive mechanisms (96, 108) of the two actuating mechanisms (84, 90) are in the form of a direct current geared motor with reversible poles, more particularly designed as a miniature motor with low power.

16. Artificial hand according to claim 15, characterised in that a coupling which can be moved angularly, more particularly a cross sliding coupling (98, 110), is disposed in each case between the drive mechanisms (96, 108) and the worm gears (94, 102) disposed downstream of the latter.

17. Artificial hand according to any one of the claims 14 to 16, characterised in that the drive shafts (92, 104) of the two control mechanisms (84, 90) are disposed in the receiving housing forming the hand body (10) so that they are essentially axially parallel to each other and are perpendicular in relation to the hand body, but the drive motors of both drive mechanisms (96, 108) are likewise disposed essentially axially parallel in relation to each other.

18. Artificial hand according to any one of the preceeding claims, characterised in that at least one revertive element operating mechanically or acoustically for example is provided in known manner for signalling the successful grasping of an object, the revertive element being at least indirectly controlled by the current which controls the actuating mechanism (96) of the fingers designed as drive motors.

**Revendications**

1. Prothèse de main comportant des doigts (18) articulés sur un corps de main (10) et constituée par au moins deux phalanges (20, 22, 24) reliées les unes aux autres par des articulations (26, 28) dont le débattement est limité par des butées (58), des câbles non extensibles (64, 66) dont au moins l'un passe du côté intérieur des doigts, dans leur direction longitudinale, et est fixé, à une extrémité, à une phalange avant (24) du doigt, un dispositif (84, 90) placé dans le corps de la main pour enrouler l'autre extrémité du câble quand les doigts se recourbent et des ressorts d'articulation (48) servant à allonger les doigts, qui sont placés sur leur dos, bandés à l'avance et extensibles, qui passent chacun au-dessus de plusieurs articulations des doigts et dont les extrémités sont fixées aux phalanges avant et au corps de main, caractérisée en ce qu'à la phalange (20) articulée sur le corps de main (10) et à l'autre phalange (22) d'un doigt (18) qui est articulée sur la phalange précédente (20) sont fixés des câbles de traction (64 et 66) dont les longueurs sont adaptées l'une à l'autre de telle façon que, quand le doigt (18) est tendu, le câble de traction (66) relié à la phalange articulée sur le corps de main (10) n'est pas sous tension.

2. Prothèse de main selon la revendication 1, caractérisée en ce que la résistance à la traction de l'élément de traction (66) servant à actionner la phalange (20) articulée sur le corps de main (10) est supérieure à celle de l'autre élément de traction (64).

3. Prothèse de main selon la revendication 1 ou 2, caractérisée en ce que les éléments de traction (64, 66) sont faits d'un câble de fibres artificielles.

4. Prothèse de main selon la revendication 1, 2 ou 3, caractérisée en ce que les phalanges (20, 22, 24) sont faites de pièces moulées, en matières plastiques dans les branches (73, 75) desquelles se trouvent des fentes (77) allant du bord de ces branches vers l'intérieur et destinées au montage d'axes (79) de rouleaux, fentes dans lesquelles peuvent être enfoncés les axes de rouleaux (79) dont les extrémités dépassent hors des rouleaux.

5. Prothèse de main selon l'une des revendications 1 à 4, caractérisée par deux dispositifs d'actionnement (84, 90) placés dans le corps de main (10) réalisés sous la forme d'un boîtier de logement et pouvant être fermés par un couvercle, en particulier un couvercle amovible, l'un de ces dispositifs servant à actionner le pouce, l'autre servant à actionner les autres doigts (18).

6. Prothèse de main selon la revendication 5, caractérisée en ce que les deux dispositifs d'actionnement (84, 90) sont réglables manuellement.

7. Prothèse de main selon la revendication 5, caractérisée en ce que les deux dispositifs d'actionnement (84, 90) peuvent être entraînés chacun par un dispositif électrique d'entraînement (96, 108).

8. Prothèse de main selon la revendication 7, caractérisée en ce que les dispositifs d'entraînement (96, 108) peuvent être commandés par myoélectricité.

9. Prothèse selon la revendication 7, caractérisée en ce que les dispositifs d'entraînement (96, 108) sont commandés par des contacts.

10. Prothèse de main selon la revendication 6 ou 7, caractérisée en ce que le dispositif d'actionnement (90) servant à actionner les doigts longs comporte les caractéristiques connues suivantes:

a) il y a pour chaque doigt long (18) un tambour d'enroulement (116) sur lequel l'élément de traction (64, 66) du doigt correspondant (18) peut être enroulé;

b) les tambours d'enroulement (116) sont placés coaxialement les uns par rapport aux autres et peuvent être mis en rotation par un élément d'entraînement commun;

c) les tambours d'enroulement (116) peuvent être tournés, contre l'action d'un ressort de rappel qui leur est fixé les uns par rapport aux autres, d'un angle de rotation qui est défini par deux butées (124, 126) pour chaque tambour d'enroulement (116), l'une de ces butées étant placée sur l'élément d'entraînement et l'autre sur le tambour d'enroulement.

11. Prothèse de main selon la revendication 10, dans laquelle le dispositif d'actionnement (90) des doigts longs peut être entraîné par un dispositif d'entraînement (96) commandé par contacts ou par un système myoélectrique, caractérisée en ce que les tambours d'enroulements (116) sont montés d'une façon connue sur un support commun, en ce que le support forme un arbre moteur (92) pouvant tourner, qui peut être entraîné, par l'intermédiaire d'un engrenage à vis sans fin (94) par le dispositif d'entraînement (96), en ce que les tambours d'enroulement (116) sont formés par des boîtiers de ressorts montés sur l'arbre moteur (92) de façon à pouvoir au moins tourner, leurs ressorts de rappel étant des ressorts moteurs en spirales montés dans ceux-ci (ressorts de remontage) dont l'une des extrémités est ancrée à la surface extérieure du boîtier de ressort et l'autre extrémité à la surface extérieure de l'arbre moteur (92) et en ce qu'il y a sur ce dernier, pour chaque boîtier de ressort, une butée (124) contre laquelle le boîtier de ressort correspondant s'applique par une contre-butée (126) sous l'effet d'une force d'appui produite par le ressort moteur.

12. Prothèse de main selon la revendication 11, caractérisée en ce que les boîtiers (116) de ressorts sont montés de façon à pouvoir être déplacés axialement sur l'arbre moteur (92) afin de bander leur ressort moteur.

13. Prothèse de main selon la revendication 11 ou 12, caractérisée en ce qu'une sécurité contre les surcharges, en particulier un accouplement réglable à friction, est intercalée entre l'engrenage à vis sans fin (94) et l'arbre moteur (92).

14. Prothèse de main selon l'une des revendications 7 à 13, caractérisée en ce que le pouce est monté sur la face extérieure du couvercle du boîtier de logement formant le corps (10) de main, en particulier de façon que la base du doigt puisse tourner et être immobilisée, et en ce que l'élément de traction (64) qui passe à travers le couvercle peut être enroulé sur un tambour d'enroulement (128) du dispositif d'actionnement (84) servant à actionner le pouce, ce dispositif d'actionnement (84) étant lui-même monté, de façon à ne pas tourner par rapport à lui, sur un arbre moteur (104) qui peut être entraîné par le dispositif électrique d'entraînement (108) au moyen d'un engrenage à vis sans fin (102) protégé par une sécurité contre les surcharges en particulier un accouplement à friction.

15. Prothèse de main selon la revendication 14, caractérisée en ce que les dispositifs d'entraînement (96, 108) des deux dispositifs d'actionnement (84, 90) sont constitués par un moteur-réducteur à courant continu dont la polarité peut être inversée, en particulier réalisé sous la forme d'un moteur micro-miniature de faible puissance.

16. Prothèse de main selon la revendication 15, caractérisée en ce qu'il y a entre les dispositifs d'entraînement (96, 108) et les engrenages à vis sans fin placés en aval (94, 102) un accouplement à débattement angulaire pour chacun, en particulier un accouplement à coulisses croisées (98, 110).

17. Prothèse selon l'une des revendications 14 à 16, caractérisée en ce que les arbres moteurs (92, 104) des deux dispositifs d'actionnement (84, 90) sont placés, dans le boîtier de logement formant le corps de main (10) dans l'ensemble avec leurs axes parallèles entre eux et perpendiculaires à celui-ci, mais que les moteurs-réducteurs des deux dispositifs d'entraînement (96, 108) sont montés également sensiblement avec leurs axes parallèles.

18. Prothèse de main selon l'une des revendications précédentes, caractérisée en ce qu'elle comporte d'une façon qui est connue, au moins un élément de signalisation en retour, à fonctionnement par exemple mécanique ou acoustique, pour signaler la prise d'un objet qui peut être commandée au moins indirectement, par le courant qui commande le dispositif d'actionnement (96) en forme de moteur-réducteur des doigts longs.

# Fig.1

Fig. 2